# EUROPEAN PATENT SPECIFICATION

(11) **EP 1 553 563 B1**
(45) Date of publication and mention of the grant of the patent: **05.11.2008**
(21) Application number: 05250125.1
(22) Date of filing: 12.01.2005
(51) Int. Cl.: G10L 19/00

(54) **Method and apparatus for converting audio data**
Verfahren und Vorrichtung zur Umwandlung von Audiodaten
Méthode et dispositif de conversion des données audio

(30) Priority: 12.01.2004 KR 2004002249
(43) Date of publication of application: 13.07.2005
(73) Proprietor: Samsung Electronics Co., Ltd., Suwon-si, Gyeonggi-Do (KR)
(72) Inventor: Dohyung, Kim, Taean-eub Hwaseong-si Gyeonggi-do (KR); Sangwook, Kim, Seocho-gu Seoul (KR); Ennmi, Oh, Seocho-gu Seoul (KR)
(74) Representative: Greene, Simon Kenneth

(56) References cited:
- EP-A- 0 918 407
- WO-A-03/096326
- US-A1- 2003 014 241
- CHI-MIN LIU, WEN-WHEI CHANG: "AUDIO CODING STANDARDS" TECHNICAL AUDIO PAPERS, 1999, pages 1-27, XP002383839 TAIWAN UNIVERSITY, www.mp3-tech.org/programmer/docs/AudioCodi ng.pdf
- BOSI M ET AL: "ISO/IEC MPEG-2 ADVANCED AUDIO CODING" JOURNAL OF THE AUDIO ENGINEERING SOCIETY, AUDIO ENGINEERING SOCIETY, NEW YORK, NY, US, vol. 45, no. 10, October 1997 (1997-10), pages 789-812, XP000730161 ISSN: 1549-4950
- HANS M ET AL: "AN MPEG AUDIO LAYERED TRANSCODER" PREPRINTS OF PAPERS PRESENTED AT THE AES CONVENTION, September 1998 (1998-09), pages 1-18, XP001014304

## Description

The present invention relates to audio data processing, and more particularly, to a method and apparatus for converting audio data compressed in a predetermined format into audio data to be compressed in another format.

An MPEG-2 layer 3 or MPEG-1 layer 3 (also known as MP3) audio devices are being gradually replaced by MPEG-4 devices with high compression efficiency. MPEG-4 is being adopted by many digital service operators such as the European digital audio broadcasting (DAB) system and Korean terrestrial digital multimedia broadcasting(DMB), in order to process video and audio signals. In particular, a bit sliced arithmetic coding (BSAC) format rather than an advanced audio coding (AAC) format is used in audio signal processing. On the other hand, an aacPlus format that combines a spectral band replication (SBR) technology with an AAC format is used as an audio signal processing technology in satellite digital multimedia broadcasting.

Meanwhile, contents including audio data compressed in an AAC format or a BSAC format have been widely used in the audio multimedia market. In addition, it is very important to provide multimedia services continuously to suit a user's taste or environment. In particular, since a plurality of devices belong to user's computing environment and various contents are used worldwide, demands for multimedia services that suit a user's taste or environment have been further increased. Here, an environment means a network or contents which a user uses. Multimedia kernel technologies for providing services suitable for a variety of environments to the user include scalability and conversion methods. In the scalability method, data is made to be suitable for a variety of environments. In the conversion method, audio data compressed in a predetermined format is converted into audio data to be compressed in another format.

In general, in the conversion method, audio input data compressed in a predetermined format is fully decoded to generate pulse coding modulation (PCM) data, and the PCM data is fully coded in a desired compression format. Accordingly, a decoding unit is needed to fully decode audio input data, and a coding unit is needed to fully code data in a desired format. Accordingly, the conversion method is expensive and time-consuming.

EP 918407 describes an audio encoding and decoding apparatus that can be used for transcoding.

A general description of audio coding standards is provided by Chi-Min Liu and Wen-Whei Chang, 'Audio Coding Standards', Technical Audio Papers, 1999, pages 1 to 27, www.mp3-tech.org/programmer/docs/AudioCoding.pdf.

The present invention provides a method of converting audio data by which audio input data compressed in a predetermined format is simply converted into audio output data to be compressed in another format depending on whether part of side information of right and left channels is shared.

The present invention also provides an apparatus for converting audio data in which audio input data compressed in a predetermined format is simply converted into audio output data to be compressed in another format depending on whether part of side information of right and left channels is shared.

According to an aspect of the present invention, there is provided a method of converting audio data according to claim 1.

According to another aspect of the present invention, there is provided an apparatus for converting audio data according to claim 10.

The above and other features and advantages of the present invention will become more apparent by describing in detail exemplary embodiments thereof with reference to the attached drawings in which:
FIG. 1 is a flowchart illustrating a method of converting audio data according to an embodiment of the present invention;
FIG. 2 is a flowchart illustrating a method of converting audio data according to another embodiment of the present invention
FIG. 3 illustrates an example of a structure of audio data compressed in an AAC format;
FIG. 4 illustrates an example of a structure of audio data compressed in a BSAC format;
FIG. 5 is a flowchart illustrating a method of converting audio data according to still another embodiment of the present invention;
FIG. 6 is a block diagram of an apparatus for converting audio data according to an embodiment of the present invention;
FIG. 7 is a block diagram of an apparatus for converting audio data according to another embodiment of the present invention; and
FIG. 8 is a block diagram of an apparatus for converting audio data according to still another embodiment of the present invention.

Hereinafter, a method and embodiments of converting audio data according to the present invention will be described in detail with reference to the attached drawings.

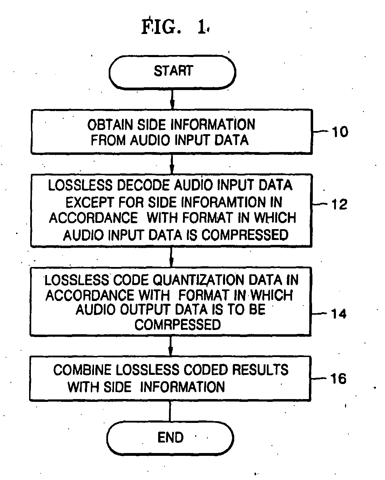

FIG. 1 is a flowchart illustrating a method of converting audio data used in an embodiment of the present invention. The method of converting audio data of FIG. 1 includes decoding audio input data (operations 10 and 12) and obtaining audio output data by coding the decoded results (operations 14 and 16).

According to the embodiment of the present invention, in operations 10 and 12, audio input data is lossless decoded in accordance with a format in which the audio input data is compressed.

For example, in operation 10, side information is obtained from the audio input data. The obtained side information may include 1-bit window_shape information, 2-bit window_sequence information, 4- or 6-bit max_sfb information, or 7-bit scale_factor_grouping information. Here, the window_shape information is information that selects one among window coefficients having a sine format or a Kaiser-Bessel derived (KBD) format. The window_sequence information is information that represents whether the type of a window used in processing one frame is long, start, short, or stop. The max_sfb information is information, which is determined according to the window_sequence information and represents a maximum of effective scalefactor bands. The scale_factor_grouping information is information, which exists only when the window_sequence information is short and represents how to group eight windows.

After operation 10, in operation 12, the audio input data except for the side information is lossless decoded in accordance with the format in which the audio input data is compressed. Here, the lossless decoded results are determined as quantized data.

After operation 12, in operations 14 and 16, the quantized data is lossless coded in accordance with a format in which audio output data is to be compressed. For example, in operation 14, the quantized data is lossless coded in accordance with the format in which the audio output data is to be compressed. After operation 14, in operation 16, the lossless coded results and the obtained side information are combined with each other, and combined results are determined as the audio output data.

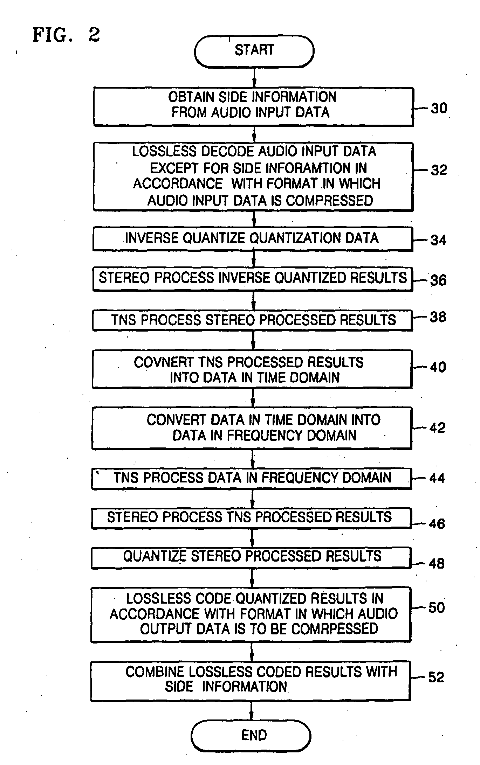

FIG. 2 is a flowchart illustrating another method of converting audio data used in the embodiment of the present invention. The method of converting audio data of FIG. 2 includes decoding audio input data (operations 30 through 40) and obtaining audio output data by coding decoded results (operations 42 through 52).

According to the present embodiment of the present invention, in operations 30 through 40, audio input data is lossless decoded in accordance with a format in which the audio input data is compressed. Operations 30 and 32 of FIG. 2 correspond to operations 10 and 12 of FIG. 1, respectively, and perform the same operations, and thus, detailed descriptions thereof will be omitted.

After operation 32, in operation 34, quantized data is inverse quantized. After operation 34, in operation 36, the inverse quantized results are stereo processed. For example, the inverse quantized results may be processed using a mid/side (M/S) stereo or an intensity stereo, etc. After operation 36, in operation 38, the stereo processed results are temporal noise shaping (TNS) processed. After operation 38, in operation 40, data in the frequency domain as the TNS processed results is converted into data in the time domain.

After operation 40, in operations 42 through 52, the data in the time domain is lossless coded in accordance with a format in which audio output data is to be compressed. For example, after operation 40, in operation 42, the data in the time domain is converted into data in the frequency domain. After operation 42, in operation 44, the data in the frequency domain is TNS processed. Here, TNS processing is to adjust quantization noise in advance using a prediction technique. After operation 44, in operation 46, the TNS processed results are stereo processed. After operation 46, in operation 48, stereo processed results are quantized. In this case, in operation 48, quantization noise can be minimized using information similar to a masking threshold value, for example, a scalefactor. Here, the information similar to the masking threshold value is a value which is not the masking threshold value but can be obtained from the masking threshold value. The information similar to the masking threshold value may be contained in the side information obtained from the audio input data. After operation 48, in operation 50, quantized results are lossless coded in accordance with the format in which the audio output data is to be compressed. After operation 50, in operation 52, lossless coded results and the obtained side information are combined with each other, and combined results are determined as the audio output data.

The method of converting audio data of FIG. 2 may include at least one of operations 34 through 40. In this case, when the method of converting audio data includes operations 40, 38, 36, and 34, operations 42, 44, 46, and 48 may be respectively included in the method of converting audio data. For example, when the method of converting audio data includes operation 34, operation 48 may be included in the method of converting audio data, and when the method of converting audio data includes operation 36, operation 46 may be included in the method of converting audio data. In addition, when the method of converting audio data includes operation 38, operation 44 may be included in the method of converting audio data, and when the method of converting audio data includes operation 40, operation 42 may be included in the method of converting audio data.

Meanwhile, a bit sliced arithmetic coding (BSAC) format, an advanced audio coding (AAC) format, or a Twin-VQ format may be used as the format in which the audio input data is compressed, or the format in which the audio output data is to be compressed. In this case, Huffman coding is used in the AAC format, and arithmetic coding is used in the BSAC format. For example, when the format in which the audio input data is compressed is the BSAC format and the format in which the audio output data is to be compressed is the AAC format, in operation 12 of FIG. 1, lossless decoding is performed using arithmetic coding, and in operation 14 of FIG. 1, lossless coding is performed using Huffman method.

In general, right and left channels have similar characteristics. Thus, part of side information of right and left channels is shared. However, in a particular case, part of the side information of the right and left channels may not be shared. When the format in which the audio input data is compressed or the format in which the audio output data is to be compressed is the BSAC format, part of the side information of the right and left channels is shared. However, when the format in which the audio input data is compressed or the format in which the audio output data is to be compressed is the AAC format, part of the side information of the right and left channels may be or not be shared.

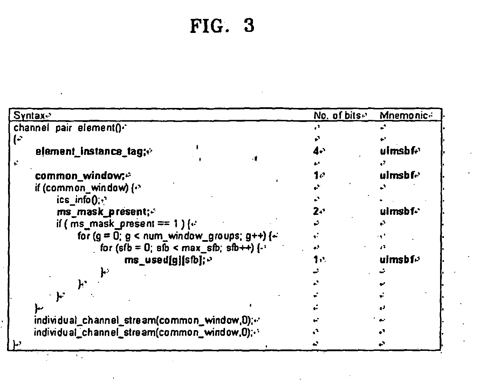

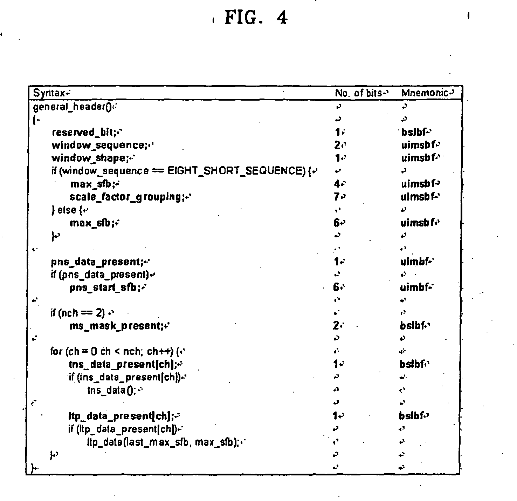

FIG. 3 illustrates an example of a structure of audio input data compressed in an AAC format or audio output data to be compressed in the AAC format. FIG. 4 illustrates an example of a structure of audio input data compressed in a BSAC format or audio output data to be compressed in the BSAC format.

As shown in FIG. 3, the audio input data compressed in the AAC format or the audio output data to be compressed in the AAC format has a 1-bit variable common_window in "channel pair element ( )". Here, the variable common_window represents whether part of side information of right and left channel is shared when audio data is a stereo.

When the variable common_window is '0', any part of the side information of the right and left channels is not shared. For example, when the variable common_window is '0', any one of window_shape information, window_sequence information, max_sfb information, or scale_factor_grouping information is not shared. However, when the variable common_window is '1', part of the side information of the right and left channels is shared. For example, when the variable common_window is '1', at least one of the window_shape information, the window_sequence information, the max_sfb information, and the scale_factor_grouping information is shared.

Contrary to this, referring to FIG. 4, the audio input data compressed in the BSAC format or the audio output data to be compressed in the BSAC format does not have the variable common_window, and part of the side information of the right and left channels is always shared.

When part of the side information of the right and left channels is shared, the audio input data is converted into the audio output data using the method of converting audio data of FIG. 1 instead of FIG. 2. For example, when the format in which the audio input data is compressed is an MPEG-4 BSAC format and the format in which the audio output data is to be compressed is an MPEG-2 or MPEG-4 AAC format, the method of converting audio data of FIG. 1 is used. Alternatively, when the format in which the audio input data is compressed is the AAC format that shares part of the side information of the right and left channels and the format in which the audio output data is to be compressed is the BSAC format, the method of converting audio data of FIG. 1 is used.

On the other hand, when any part of the side information of the right and left channels is not shared, the audio input data is converted into the audio output data using the method of converting audio data of FIG. 2 instead of FIG. 1. In this case, when decoded results are coded in operations 42 through 52 of FIG. 2, either the side information of the left channel or the side information of the right channel is used. In this case, the use of the side information of the left channel or the side information of the right channel may be determined according to the use purpose of side information. For example, when window_sequence in the side information of the left channel is long and window_sequence in the side information of right channel is short, the use of the side information of the left channel or the side information of the right channel is determined according to the use purpose of side information. Here, even though any side information is determined, the case the variable common_window is '1' based on the entire frame is rare. Thus, a kind of the determined side information has a little effect on the method of converting audio data according to the present invention. For example, when the format in which the audio input data is compressed is the MPEG-2 or MPEG-4 AAC format in which any part of the side information of the right and left channels is not shared and the format in which the audio output data is to be compressed is the MPEG-4 BSAC format, the audio input data can be converted into the audio output data using the method of converting audio data of FIG. 2.

Meanwhile, whether part of the side information of the right and left channels is shared may be determined according to each frame. Thus, the method of converting audio data of FIG. 1 or 2 may be differently applied according to each frame.

According to an embodiment of the present invention, the method of converting audio data of FIG. 2 may be performed from a current frame until a frame in which part of the side information of the right and left channels is shared appears.

According to another embodiment of the present invention, the method of converting audio data of FIG. 2 may be performed from the previous frame of the current frame until a frame in which part of the side information of the right and left channels is shared appears. The main reason why the side information of the left channel is different from the side information of the right channel is that the window_sequence information of the left channel is different from that of the right channel. That is, one channel of the right and left channels uses a long window, and the other channel thereof uses a short window. In this case, since the audio input data processed using the long window cannot immediately be converted into the audio output data processed using the short window, in general, the audio input data processed using the long window is converted into the audio output data processed using a start window, and then, the audio input data processed using the start window is converted into the audio output data processed using the short window. Thus, it is preferable that the audio input data may be converted into the audio output data in consideration of the previous frame, because of overlap and add features in which half of the previous frame and half of the current frame are overlapped and processed and which appear when inverse modified discrete cosine transform (IMDCT) is performed.

First, as shown in Table 1, it is assumed that the audio input data compressed in the AAC format having a different bit in each frame is converted into the audio output data to be compressed in the BSAC format.

**Table 1**

| | | | | | | | |
|---|---|---|---|---|---|---|---|
| Classifications | Channels | Frame 1 | Frame 2 | Frame 3 | Frame 4 | Frame 5 | Frame 6 |
| | Right channel | 0 | 0 | 0 | 0 | 0 | 0 |
| | Left channel | 0 | 1 | 2 | 3 | 0 | 0 |

As shown in Table 1, it is assumed that a variable common_window in a frame 1 is '1', a variable common_window from a frame 2 to a frame 4 is '0' and a variable common_window from a frame 5 to a frame 6 is '1'.

On these assumption, according to an embodiment of the present invention, the method of converting audio data of FIG. 1 may be applied to the previous frame (frame 1), and the method of converting audio data of FIG. 2 may be applied to from the current frame (frame 2), to a frame (frame 5) in which part of the side information of the right and left channels is shared appears, that is, to a frame (frame 4).

According to another embodiment of the present invention, even though the method of converting audio data of FIG. 1 is applied to the previous frame (frame 1), the method of converting audio data of FIG. 2 may be applied to from the previous frame (frame 1) of the current frame (frame 2), to a frame (frame 5) in which part of the side information of the right and left channels is shared appears, that is, a frame (frame 4), when converting the current frame (frame 2).

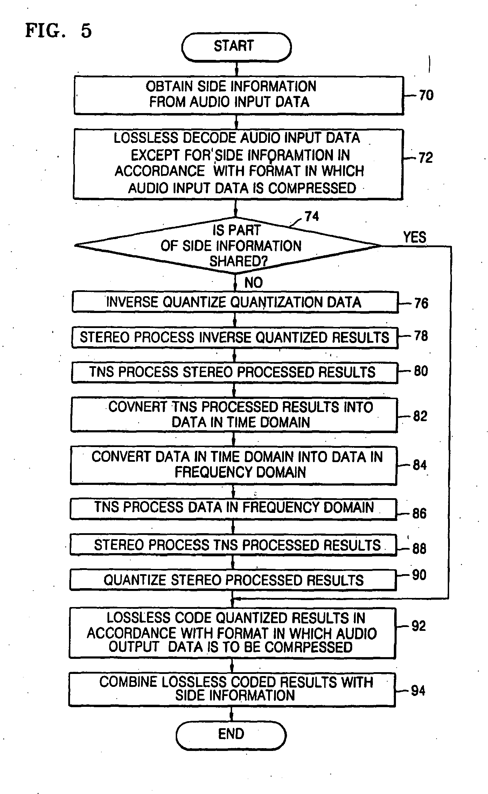

FIG. 5 is a flowchart illustrating a method of converting audio data according to still another embodiment of the present invention. The method of converting audio data of FIG. 5 includes decoding audio input data (operations 70 through 82) and obtaining audio output data by coding decoded results (operations 84 through 94).

Operations 70 and 72 of FIG. 5 correspond to operations 30 and 32 of FIG. 2, respectively, and performs the same operations, and thus, detailed descriptions thereof will be omitted. In addition, operations 76 through 94 of FIG. 5 correspond to operations 34 through 52 of FIG. 2, respectively, and performs the same operations, and thus, detailed descriptions thereof will be omitted. Consequently, the method of converting audio data of FIG. 5 is the same as the method of converting audio data of FIG. 2 excluding that the method of FIG. 5 further includes operation 74.

According to the present embodiment of the present invention, in operation 74, it is determined whether part of side information of right and left channels is shared.

If it is determined that any part of the side information of the right and left channels is not shared, the method proceeds to operation 76. In this case, in the method of converting audio data of FIG. 5 like in the method of converting audio data of FIG. 2, operations 76 through 94 are performed to generate converted audio output data. In this case, the method of converting audio data of FIG. 5 may further include at least one of operations 76, 78, 80, and 82 like in the method of converting audio data of FIG. 2. In this case, when the method of converting audio data of FIG. 5 includes operations 76, 78, 80, and 82, operations 90, 88, 86, and 84 may be included in the method of converting audio data of FIG. 5.

However, if it is determined that part of the side information of the right and left channels is shared, the method proceeds to operation 92. In this case, in the method of converting audio data of FIG. 5 like in the method of converting audio data of FIG. 1, operations 14 and 16 are performed to generate converted audio output data.

Hereinafter, an apparatus for converting audio data according to the present invention will be described in detail with reference to the attached drawings.

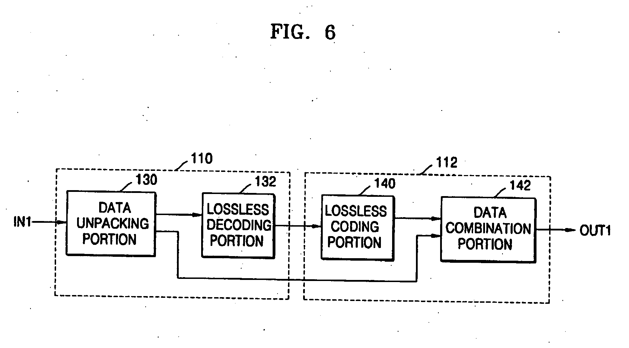

FIG. 6 is a block diagram of an apparatus for converting audio data according to an embodiment of the present invention. The apparatus for converting audio data of FIG. 6 includes a decoding unit 110 and a coding unit 112.

The decoding unit 110 lossless decodes audio input data in accordance with a format in which audio input data input through an input terminal IN1 is compressed, and outputs lossless decoded results to the coding unit 112.

In this case, the coding unit 112 lossless codes the lossless decoded results input from the coding unit 110 in accordance with a format in which audio output data is to be compressed, and outputs lossless coded results to an output terminal OUT1.

According to the present embodiment of the present invention, the decoding unit 110 and the coding unit 112 may be implemented as shown in FIG. 6. That is, the decoding unit 110 may include a data unpacking portion 130 and a lossless decoding portion 132, and the coding unit 112 may include a lossless coding portion 140 and a data combination portion 142. In this case, the apparatus for converting audio data of FIG. 6 may also perform the method of converting audio data of FIG. 1.

In order to perform operation 10, the data unpacking portion 130 obtains side information by unpacking the audio input data having a bit stream pattern input through the input terminal IN1, outputs the obtained side information to the data combination portion 142, and outputs the audio input data excluding the side information to the lossless decoding portion 132.

In order to perform operation 12, the lossless decoding portion 132 inputs the audio input data except for the side information , from the data unpacking portion 130, lossless decodes the audio input data except for the side infomation in accordance with the format in which the audio input data is compressed, and outputs lossless decoded results as quanitization data to the coding unit 112. For example, when the compressed format of the audio input data is a bit sliced arithmetic coding (BSAC) format, the lossless decoding portion 132 performs lossless decoding using arithmetic method. However, when the compressed format of the audio input data is an advanced audio coding (AAC) format, the lossless decoding portion 132 performs lossless decoding using Huffman method.

In order to perform operation 14, the lossless coding portion 140 lossless codes the quantized data input from the lossless decoding portion 132 in accordance with the format in which the audio output data is to be compressed, and outputs lossless coded results to the data combination portion 142. For example, when the format in which the audio output data is to be compressed is a BSAC format, the lossless coding portion 140 performs lossless coding using arithmetic coding. However, when the format in which the audio output data is to be compressed is an AAC format, the lossless coding portion 140 performs lossless coding using Huffman coding.

In order to perform operation 16, the data combination portion 142 combines the lossless coded results obtained by the lossless coding portion 140 with the side information input from the data unpacking portion 130 and outputs combined results as the audio output data to an output terminal OUT1.

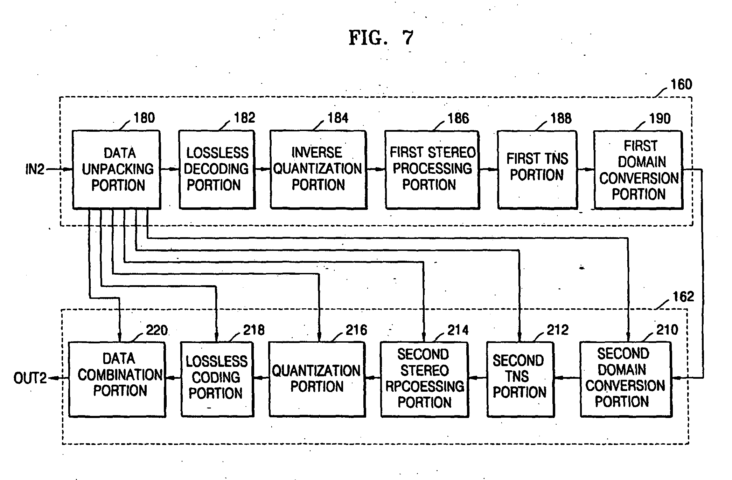

FIG. 7 is a block diagram of an apparatus for converting audio data according to another embodiment of the present invention. The apparatus of FIG. 7 includes a decoding unit 160 and a coding unit 162. The decoding unit 160 and the coding unit 162 of FIG. 7 performs the same operations as those of the decoding unit 110 and the coding unit 112 of FIG. 6.

According to the present embodiment of the present invention, as shown in FIG. 7, the decoding unit 160 may include a data unpacking portion 180, a lossless decoding portion 182, an inverse quantization portion 184, a first stereo processing portion 186, a first temporal noise shaping (TNS) portion 188, and a first domain conversion portion 190. In addition, the coding unit 162 may include a second domain conversion portion 210, a second TNS portion 212, a second stereo processing portion 214, a quantization portion 216, a lossless coding portion 218, and a data combination portion 220. In this case, the apparatus for converting audio data of FIG. 7 may perform the method of converting audio data of FIG. 2.

The data unpacking portion 180 and the lossless decoding portion 182 of FIG. 7 which respectively perform operations 30 and 32 of FIG. 2, performs the same operations as those of the data unpacking portion 130 and the lossless decoding portion 132 of FIG. 6, and thus, detailed descriptions thereof will be omitted.

In order to perform operation 34, the inverse quantization portion 184 inverse quantizes the quantized data input from the lossless decoding portion 182 and outputs inverse quantized results to the first stereo processing portion 186.

In order to perform operation 36, the first stereo processing portion 186 stereo processes the inverse quantized results obtained by the inverse quantization portion 184 and outputs stereo processed results to the first TNS portion 188.

In order to perform operation 38, the first TNS portion 188 TNS processes the stereo processed results obtained by the first stereo processing portion 186 and outputs TNS processed results to the first domain conversion portion 190.

In order to perform operation 40, the first domain conversion portion 190 converts data in the frequency domain as the TNS processed results obtained by the first TNS portion 188 into data in the time domain and outputs the data in the time domain to the coding unit 162.

In order to perform operation 42, the second domain conversion portion 210 converts the data in the time domain input from the first domain conversion portion 190 into data in the frequency domain and outputs the converted data in the frequency domain to the second TNS portion 212.

In order to perform operation 44, the second TNS portion 212 TNS processes the data in the frequency domain input from the second domain conversion portion 210 and outputs TNS processed results to the second stereo processing portion 214.

In order to perform operation 46, the second stereo processing portion 214 stereo processes the TNS processed results obtained by the second TNS portion 212 and outputs stereo processed results to the quantization portion 216.

In order to perform operation 48, the quantization portion 216 quantizes the stereo processed by the second stereo processing portion 214 and outputs quantized results to the lossless coding portion 218. In this case, the quantization portion 216 can minimize quantization noise using information, which is contained in the obtained side information input from the data unpacking portion 180 and which is similar to a masking threshold value. In a conventional conversion method, a separate auditory psychological sound modeling unit which calculates a masking threshold value from the side information contained in the audio input data, should be provided, and quantization noise is minimized using the calculated masking threshold value. Thus, due to the separate auditory psychological sound modeling unit, costs increase.

In order to perform operation 50, the lossless coding portion 218 lossless codes the quantized results obtained by the quantization portion 216 in accordance with the format in which the audio output data is to be compressed and outputs lossless coded results to the data combination portion 220.

In order to perform operation 52, the data combination portion 220 combines the lossless coded results with the side information input from the data unpacking portion 180 and outputs combined results as the audio output data to an output terminal OUT2.

The coding unit 162 of FIG. 7 codes the decoded results obtained by the decoding unit 160 using only side information of one channel of right and left channels. For example, the second domain conversion portion 210, the second TNS portion 212, the second stereo processing portion 214, the quantization portion 216, the lossless coding portion 218, and the data combination portion 220 of the coding unit 162, which input the side information output from the data unpacking portion 180, perform coding using only side information of one channel of right and left channels.

The decoding unit 160 of FIG. 7 may include at least one of the inverse quantization portion 184, the first stereo processing portion 186, the first TNS portion 188, and the first domain conversion portion 190. Similarly, the coding unit 162 may include at least one of the second domain conversion portion 210, the second TNS portion 212, the second stereo processing portion 214, and the quantization portion 216. If the decoding unit 160 of FIG. 7 includes the first domain conversion portion 190, the first TNS portion 188, the first stereo processing portion 186, and the inverse quantization portion 184, the coding unit 162 may include the second domain conversion portion 210, the second TNS portion 212, the second stereo processing portion 214, and the quantization portion 216.

The apparatus for converting audio data of FIG. 6 is used when part of the side information of the right and left channels is shared, and the apparatus for converting audio data of FIG. 7 is used when any part of the side information of the right and left channels is not shared.

Meanwhile, whether part of the side information of the right and left channels is shared may be determined differently according to each frame. Thus, the apparatus for converting audio data of FIG. 6 or 7 may be applied to each frame.

Here, the apparatus for converting audio data of FIG. 7 may be applied to from the previous frame of a current frame until a frame in which part of the side information of the right and left channels is shared appears, so as to convert the audio input data into the audio output data. Alternatively, the apparatus for converting audio data of FIG. 7 may be applied to from the current frame until a frame in which part of the side information of the right and left channels is shared appears, so as to convert the audio input data into the audio output data.

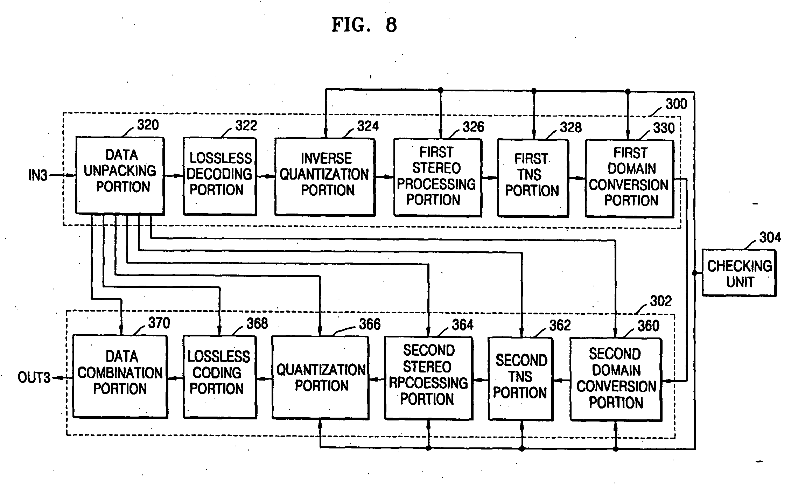

FIG. 8 is a block diagram of an apparatus for converting audio data according to still another embodiment of the present invention. The apparatus for converting audio data of FIG. 8 includes a decoding unit 300, a coding unit 302, and a checking unit 304.

The decoding unit 300 and the coding unit 302 of FIG. 8 perform the same operations as those of the decoding unit 110 and the coding unit 112 of FIG. 6.

According to the present embodiment of the present invention, as shown in FIG. 8, the decoding unit 300 may include a data unpacking portion 320, a lossless decoding portion 322, an inverse quantization portion 324, a first stereo processing portion 326, a first temporal noise shaping (TNS) portion 328, and a first domain conversion portion 330. In addition, the coding unit 302 may include a second domain conversion portion 360, a second TNS portion 362, a second stereo processing portion 364, a quantization portion 366, a lossless coding portion 368, and a data combination portion 370. In this case, the apparatus for converting audio data of FIG. 8 may perform the method of converting audio data of FIG.5.

The apparatus for converting audio data of FIG. 8 is the same as the apparatus for converting audio data of FIG. 7 excluding that the apparatus of FIG. 8 further includes a checking unit 304 and each of the decoding unit 300 and the coding unit 302 is operated using checked results of the checking unit 304. Thus, only a difference between the apparatus for converting audio data of FIG. 8 and the apparatus for converting audio data of FIG. 7 will now be described.

In order to perform operation 74, the checking unit 304 checks whether part of side information of right and left channels is shared, and outputs checking results to each of the decoding unit 300 and the coding unit 302. In this case, if it is recognized in response to checked results of the checking unit 304, that is, from the checked results, that part of the side information of the right and left channels is shared, the inverse quantization portion 324, the first stereo processing portion 326, the first temporal noise shaping (TNS) portion 328, the first domain conversion portion 330, the second domain conversion portion 360, the second TNS portion 362, the second stereo processing portion 364, and the quantization portion 366 operate.

As described above, in the method and apparatus for converting audio data according to the present invention, when part of side information of right and left channels is shared, full decoding and full coding are not performed, and as shown in FIG. 1 or 6, audio input data is simply converted into audio output data. Thus, costs are reduced and a conversion speed increases. Even when any part of the side information of the right and left channels is not shared, as shown in FIG. 2, 5, 7 or 8, the audio input data is simply converted into the audio input data in comparison with the conventional conversion method, that is, a separate auditory psychological sound modeling unit (not shown) is not needed. Thus, costs are reduced and a conversion speed increases. Accordingly, multimedia services are seamlessly provided to suit a user's taste or environment in various applications, and the user can use fast and various contents formats when using an advanced audio coding (AAC) format and a bit-sliced arithmetic coding (BSAC) format together for compression of audio data. For example, in a home network environment, when digital broadcasting received from outside to house is transmitted to a device inside the house via a home gateway, audio input data can be easily converted into audio output data to suit a compression format of the device to which broadcasting is transmitted such that desired services are seamlessly provided to any device inside the house.

While the present invention has been particularly shown and described with reference to exemplary embodiment thereof, it will be understood by those skilled in the art that various changes in form and details may be made therein without departing from the scope of the invention as defined by the following claims.

## Claims

1. A method of converting audio data, the method comprising:
lossless decoding audio input data in accordance with a format in which the audio input data is compressed by:
obtaining side information (10,30) from the audio input data; and
checking (74) whether any part of the side information of the right and left channels is not shared to determine a determination result,
lossless decoding (12,32) the audio input data except for the side information in accordance with the format in which the audio input data is compressed and determining the lossless decoded results as quantized data; and
whether or not any part of the side information is shared:
lossless coding (14, 50) the quantized data in accordance with the format in which the audio output data is to be compressed; and
combining (16, 52) the lossless coded results with the obtained side information to generate the audio output data;
wherein, if and only if any part of the side information of the right and left channels is not shared, the lossless decoding of audio input data comprises the operations of:
(a1) inverse quantizing (34) the quantized data;
(a2) stereo processing (36) the inverse quantized results;
(a3) temporal noise shaping (TNS) processing 38 the stereo processed results;
and
(a4) converting data (40) in a frequency domain as the TNS processed results into data in the time domain,
and the lossless coding of lossless decoded results comprises the operations of:
(b1) converting (42) the data in the time domain into the data in the frequency domain;
(b2) TNS processing (44) the data in the frequency domain;
(b3) stereo processing (46) the TNS processed results; and
(b4) quantizing (48) the stereo processed results; and
wherein the decoded results are coded using only side information of one channel of the right and left channels.

2. The method of claim 1 wherein in operation (b4), quantization noise is minimized using information contained in the side information obtained from the audio input data and similar to a masking threshold value.

3. The method of claim 1, wherein the method performed according to each frame is performed from a previous frame of a current frame until a frame in which part of the side information of the right and left channels is shared appears.

4. The method of claim 1, wherein the method performed according to each frame is performed from a current frame until a frame in which part of the side information of the right and left channels is shared appears.

5. The method of any of claims 1 to 6, wherein the format in which the audio input data is compressed is a bit sliced arithmetic coding (BSAC) format, and the format in which the audio output data is to be compressed is an advanced audio coding (AAC) format.

6. The method of any of claims 1 to 6, wherein the format in which the audio input data is compressed is an advanced audio coding (AAC) format, the format in which the audio output data is to be compressed is a bit sliced arithmetic coding (BSAC) format, and the advanced audio coding (AAC) format shares part of the side information of the right and left channels.

7. The method of claim 5 or 6 wherein a standard to which the AAC format belongs is one of an MPEG-2 standard or MPEG-4 standard.

8. The method of claim 5, 6, or 7, wherein a standard to which the BSAC format belongs is an MPEG-4 standard.

9. The method of claim 1, wherein the format in which the audio input data is compressed is an advanced audio coding (AAC) format, the format in which the audio output data is to be compressed is a bit sliced arithmetic coding (BSAC) format, and the advanced audio coding (AAC) format does not share any part of the side information of the right and left channels.

10. An apparatus for converting audio data, the apparatus comprising:
a decoding unit (300) arranged to lossless decode audio input data in accordance with a format in which the audio input data is compressed; and
a coding unit (302) arranged to lossless code lossless decoded results in accordance with a format in which audio output data is to be compressed.
wherein the decoding unit (300) comprises:
a data unpacking portion (320) arranged to obtain side information from the audio input data; and
a lossless decoding portion (322) arranged to lossless decode the audio input data except for the side information in accordance with the format in which the audio input data is compressed and outputting lossless decoded results as quantized data,
wherein the coding unit (302) comprises:
a lossless coding portion (368) arranged to lossless code the quantized data in accordance with the format in which the audio output data is to be compressed; and
a data combination portion (370) arranged to combine lossless coded results with the side information to generate the audio output data.
wherein the decoding unit further comprises at least one of:
an inverse quantization portion (324) arranged to inverse quantize the quantized data input from the lossless decoding unit;
a first stereo processing portion (326) stereo arranged to process the inverse quantized results;
a first temporal noise shaping (TNS) portion (328) TNS processing the stereo processed results; and
a first domain conversion portion (330) arranged to convert data in frequency domain as TNS processed results into data in time domain,
wherein the coding unit (302) further comprises at least one of:
a second domain conversion portion (360) arranged to convert the data in the time domain into data in the frequency domain;
a second TNS portion (362) TNS arranged to process the data in the frequency domain;
a second stereo processing portion (364) arranged to stereo process the TNS processed results; and
a quantization portion (366) arranged to quantize the stereo processed results obtained by the second stereo processing portion and arranged to output quantized results to the lossless coding portion,
wherein the lossless coding portion (368) is arranged to lossless code the quantized results obtained by the quantization portion in accordance with the format in which the audio output data is to be compressed, and
**characterized by** further comprising a checking unit arranged to determine whether part of the side information of the right and left channels is shared and outputting the determination result,
wherein in response to checked results, the at least one of the inverse quantization portion (324), the first stereo processing portion (326), the first TNS portion (328), the first domain conversion portion (330), and the at least one of the second domain conversion portion (360), the second TNS portion (362), the second stereo processing portion (364), and the quantization portion (366) are arranged to operate depending on the checked results;
and in that only when any part of the side information of the right and left channels is not shared, the first domain conversion portion (330), the first TNS portion (328), the first stereo processing portion (326) and the inverse quantization portion (324), the second domain conversion portion (360), the second TNS portion (362), the second stereo processing portion (364), and the quantization portion (366), are arranged to operate;
wherein the coding unit (302) codes the decoded results using only side information of one channel of the right and left channels.

11. The apparatus of claim 10, wherein the quantization portion (366) is arranged to minimize quantization noise using information contained in the side information obtained from the audio input data and similar to a masking threshold value.

12. The apparatus of claim 10, wherein the apparatus operating according to each frame is arranged to operate from a previous frame of a current frame until a frame in which part of the side information of the right and left channels is shared appears.

13. The apparatus of claim 10, wherein the apparatus operating according to each frame is arranged to operate from a current frame until a frame in which part of the side information of the right and left channels is shared appears.

## Patentansprüche

1. Verfahren zum Konvertieren von Audiodaten, das die folgenden Schritte beinhaltet:
verlustloses Decodieren von Audioeingangsdaten mit einem Format, in dem die Audioeingangsdaten kompromiert werden, durch:
Einholen von Seiteninformatioen (10, 30) von den Audioeingangsdaten; und
Prüfen (74), ob irgendein Teil der Seiteninformationen des rechten und linken Kanals nicht gemeinsam genutzt wird, um ein Ermittlungsergebnis zu erhalten,
verlustloses Decodieren (12, 32) der Audioeingangsdaten, die Seiteninformationen ausgenommen, gemäß dem Format, in dem die Audioeingangsdaten komprimiert werden, und Bestimmen der verlustlos decodierten Ergebnisse als quantisierte Daten; und
unabhängig davon, ob irgendein Teil der Seiteninformationen gemeinsam genutzt wird:
verlustloses Codieren (14, 50) der quantisierten Daten gemäß dem Format, in dem die Audioausgangsdaten komprimiert werden sollen; und
Kombinieren (16, 52) der verlustlos codierten Ergebnisse mit den erhaltenen Seiteninformationen, um die Audioausgangsdaten zu erzeugen;
wobei nur dann, wenn irgendein Teil der Seiteninformationen des rechten und linken Kanals nicht gemeinsam genutzt wird, das verlustlose Decodieren von Audioeingangsdaten die folgenden Operationen beinhaltet:
a1) inverses Quantisieren (34) der quantisierten Daten;
a2) Stereoverarbeiten (36) der invers quantisierten Ergebnisse;
a3) TNS-(Temporal Noise Shaping = Temporale Geräuschformung)-Verarbeiten (38) der stereo verarbeiteten Ergebnisse; und
a4) Konvertieren von Daten (40) in einer Frequenzdomäne als die TNS-verarbeiteten Ergebnisse in Daten in der Zeitdomäne,
und wobei das verlustlose Codieren von verlustlos decodierten Ergebnissen die folgenden Operationen beinhaltet:
b1) Konvertieren (42) der Daten in der Zeitdomäne in die Daten in der Frequenzdomäne;
b2) TNS-Verarbeiten (44) der Daten in der Frequenzdomäne;
b3) Stereoverarbeiten (46) der TNS-verarbeiteten Ergebnisse; und
b4) Quantisieren (48) der Stereo verarbeiteten Ergebnisse; und
wobei die decodierten Ergebnisse nur mittels Seiteninformationen von einem aus dem rechten und dem linken Kanal codiert werden.

2. Verfahren nach Anspruch 1, wobei in Operation b4 Quantisierungsrauschen mittels Informationen minimiert wird, die in den von den Audioeingangsdaten erhaltenen Seiteninformationen enthalten sind und die einem Maskierungsschwellenwert ähnlich sind.

3. Verfahren nach Anspruch 1, wobei das an den einzelnen Frames ausgeführte Verfahren von einem dem aktuellen Frame vorangehenden Frame bis zu einem Frame ausgeführt wird, in dem ein Teil der Seiteninformationen des rechten und linken Kanals gemeinsam genutzt wird.

4. Verfahren nach Anspruch 1, wobei das an den einzelnen Frames ausgeführte Verfahren von einem aktuellen Frame bis zu einem Frame ausgeführt wird, in dem ein Teil der Seiteninformationen des rechten und linken Kanals gemeinsam genutzt wird.

5. Verfahren nach einem der Ansprüche 1 bis 6, wobei das Format, in dem die Audioeingangsdaten komprimiert werden, ein BSAC-(Bit-sliced Arithmetic Coding = Audiokomprimierung bei MPEG-4)-Format ist, und das Format, in dem die Audioausgangsdaten komprimiert werden sollen, ein AAC-(Advanced Audio Coding = Verbesserte Audiocodierungstechnik)-Format ist.

6. Verfahren nach einem der Ansprüche 1 bis 6, wobei das Format, in dem die Audioeingangsdaten komprimiert sind, ein AAC-(Advanced Audio Coding)-Format ist, und das Format, in dem die Audioausgangsdaten komprimiert werden sollen, ein BSAC-(Bit-sliced Arithmetic Coding)-Format ist, und das AAC-(Advanced Audio Coding)-Format einen Teil der Seiteninformationen des rechten und linken Kanals gemeinsam nutzt.

7. Verfahren nach Anspruch 5 oder 6, wobei der Standard, zu dem das AAC-Format gehört, entweder der MPEG-2 Standard oder der MPEG-4 Standard ist.

8. Verfahren nach Anspruch 5, 6 oder 7, wobei der Standard, zu dem das BSAC-Format gehört, der MPEG-4 Standard ist.

9. Verfahren nach Anspruch 1, wobei das Format, in dem die Audioeingangsdaten komprimiert wird, ein AAC-(Advanced Audio Coding)-Format ist, das Format, in dem die Audioausgangsdaten komprimiert werden sollen, ein BSAC-(Bit-sliced Arithmetic Coding)-Format ist, und das AAC-(Advanced Audio Coding)-Format keine Teil der Seiteninformationen des rechten und linken Kanals gemeinsam nutzt.

10. Vorrichtung zum Konvertieren von Audiodaten, wobei die Vorrichtung Folgendes umfasst:
eine Decodiereinheit (300) zum verlustlosen Decodieren von Audioeingangsdaten mit einem Format, in dem die Audioeingangsdaten komprimiert werden; und
eine Codiereinheit (302) zum verlustlosen Codieren von verlustlos decodierten Ergebnissen mit einem Format, in dem Audioausgangsdaten komprimiert werden sollen;
wobei die Decodiereinheit (300) Folgendes umfasst:
einen Datenauspackteil (320) zum Gewinnen von Seiteninformationen von den Audioeingangsdaten; und
einen Verlustlos-Decodieren-Teil (322) zum verlustlosen Decodieren der Audioeingangsdaten, die Seiteninformationen ausgenommen, gemäß dem Format, in dem die Audioeingangsdaten komprimiert werden, und zum Ausgeben von verlustlos decodierten Ergebnissen als quantisierte Daten;
wobei die Codiereinheit (302) Folgendes umfasst:
einen Verlustlos-Codieren-Teil (368) zum verlustlosen codieren der quantisierten Daten gemäß dem Format, in dem die Audioausgangsdaten komprimiert werden sollen; und
einen Datenkombinationsteil (370) zum Kombinieren von verlustlos codierten Ergebnissen mit den Seiteninformationen, um die Audioausgangsdaten zu erzeugen;
wobei die Decodiereinheit ferner wenigstens eines der Folgenden umfasst:
einen Invers-Quantisieren-Teil (324) zum inversen Quantisieren der von der Verlustlos-Decodieren-Einheit eingegebenen quantisieren Daten;
einen ersten Stereoverarbeitungsteil (326), stereo angeordnet zum Verarbeiten der invers quantisierten Ergebnisse;
einen TNS-(Temporal Noise Shaping)-Teil (328) zum TNS-Verarbeiten der stereo verarbeiteten Ergebnisse; und
einen ersten Domänenkonvertierungsteil (330) zum Konvertieren von Daten in der Frequenzdomäne als TNSverarbeitete Ergebnisse in Daten in der Zeitdomäne,
wobei die Codiereinheit (302) ferner wenigstens eines der Folgenden umfasst:
einen zweiten Domänenkonvertierungsteil (360) zum Konvertieren der Daten in der Zeitdomäne in Daten in der Frequenzdomäne;
einen zweiten TNS-Teil (362), TNS-angeordnet zum Verarbeiten der Daten in der Frequenzdomäne;
einen zweiten Stereoverarbeitungsteil (364) zum Stereoverarbeiten der TNS-verarbeiteten Ergebnisse; und
einen Quantisierungsteil (366) zum Quantisieren der vom zweiten Stereoverarbeitungsteil erhaltenen stereoverarbeiteten Ergebnisse und zum Ausgeben von quantisierten Ergebnissen an den Verlustlos-Codieren-Teil;
wobei der Verlustlos-Codieren-Teil (368) die Aufgabe hat, die vom Quantisierungsteil erhaltenen quarzitisierten Ergebnisse gemäß dem Format verlustlos zu codieren, in dem die Audioausgabedaten komprimiert werden sollen, und
**dadurch gekennzeichnet, dass** sie ferner eine Prüfeinheit mit der Aufgabe umfasst zu ermitteln, ob ein Teil der Seiteninformationen des rechen und linken Kanals gemeinsam genutzt wird, und das Ermittlungsergebnis auszugeben,
wobei als Reaktion auf geprüfte Ergebnisse der wenigstens eine aus Invers-Quantisieren-Teil (324), erster Stereoverarbeitungsteil (326), erster TNS-Teil (328), erster Domänenkonvertierungsteil (330), und der wenigstens eine aus zweiter Domänenkonvertierungsteil (360), zweiter TNS-Teil (362), zweiter Stereoverarbeitungsteil (364) und Quantisierungsteil (366) die Aufgabe haben, je nach den geprüften Ergebnissen zu arbeiten;
und dadurch, dass nur dann, wenn irgendein Teil der Seiteninformationen des rechten und linken Kanals nicht gemeinsam genutzt wird, der erste Domänenkonvertierungsteil (330), der erste TNS-Teil (328), der erste Stereoverarbeitungsteil (326) und der Invers-Quantisieren-Teil (324), der zweite Domänenkonvertierungsteil (360), der zweite TNS-Teil (362), der zweite Stereoverarbeitungteil (364) und der Quantisierungsteil (366) arbeiten können,
wobei die Codiereinheit (302) die decodierten Ergebnisse nur mit Seiteninformationen von einem Kanal aus dem rechten und dem linken Kanal codiert.

11. Vorrichtung nach Anspruch 10, wobei der Quantisierungsteil (366) die Aufgabe hat, Quantisierungsrauschen mittels Informationen zu minimieren, die in den von den Audioeingangsdaten erhaltenen Seiteninformationen enthalten sind und die einem Maskierungsschwellenwert ähnlich sind.

12. Vorrichtung nach Anspruch 10, wobei die gemäß jedem Frame arbeitende Vorrichtung die Aufgabe hat, von einem dem aktuellen Frame vorangehenden Frame bis zu einem Frame zu arbeiten, in dem ein Teil der Seiteninformationen des rechten und linken Kanals gemeinsam genutzt wird.

13. Vorrichtung nach Anspruch 10, wobei die gemäß jedem Frame arbeitende Vorrichtung die Aufgabe hat, von einem aktuellen Frame bis zu einem Frame zu arbeiten, in dem ein Teil der Seiteninformationen des rechten und linken Kanals gemeinsam genutzt wird.

## Revendications

1. Procédé de conversion de données audio comprenant :
le décodage sans perte de données d'entrée audio conformément à un format dans lequel les données d'entrée audio sont comprimées en :
obtenant des informations latérales (10, 30) des données d'entrée audio ; et
vérifiant (74) si une partie des informations latérales des canaux droit et gauche n'est pas partagée pour déterminer un résultat de détermination,
décodant sans perte (12, 32) les données d'entrée audio à l'exception des informations latérales conformément au format dans lequel les données d'entrée audio sont comprimées et en déterminant les résultats décodés sans perte en tant que données quantifiées ; et
qu'une partie des informations latérales soit ou non partagée :
en codant sans perte (14, 50) les données quantifiées conformément au format dans lequel les données de sortie audio doivent être comprimées ; et
en combinant (16, 52) les résultats codés sans perte avec les informations latérales obtenues pour générer les données de sortie audio ;
dans lequel, si et seulement si une partie des informations latérales des canaux droit et gauche ne sont pas partagées, le décodage sans perte des données d'entrée audio comprend les opérations suivantes :
(a1) quantification inverse (34) des données quantifiées ;
(a2) traitement stéréo (36) des résultats de quantification inverse ;
(a3) traitement par mise en forme temporelle du bruit (TNS) (38) des résultats du traitement stéréo ;
et
(a4) conversion des données (40) situées dans un domaine fréquence en tant que résultats du traitement TNS en données dans le domaine temps,
et le codage sans perte des résultats décodés sans perte comprend les opérations de :
(b1) conversion (42) des données situées dans le domaine temps en données dans le domaine fréquence ;
(b2) traitement TNS (44) des données situées dans le domaine fréquence ;
(b3) traitement stéréo (46) des résultats du traitement TNS ; et
(b4) quantification (48) des résultats du traitement stéréo ; et
dans lequel les résultats décodés sont codés en utilisant seulement les informations latérales de l'un des canaux droit et gauche.

2. Procédé selon la revendication 1, dans lequel, dans l'opération (b4), la quantification du bruit est minimisée en utilisant les informations contenues dans les informations latérales obtenues des données d'entrée audio et similaires à une valeur seuil de masquage.

3. Procédé selon la revendication 1, dans lequel le procédé exécuté conformément à chaque trame est exécuté à partir d'une trame précédente d'une trame actuelle jusqu'à ce qu'apparaisse une trame dans laquelle une partie des informations latérales des canaux droit et gauche est partagée.

4. Procédé selon la revendication 1, dans lequel le procédé exécuté selon chaque trame est exécuté à partir d'une trame actuelle jusqu'à ce qu'apparaisse une trame
dans laquelle une partie des informations latérales des canaux droit et gauche est partagée.

5. Procédé selon l'une quelconque des revendications 1 à 6, dans lequel le format dans lequel les données d'entrée audio sont comprimées est un format de codage arithmétique à bit tranché (BSAC), et le format dans lequel les données de sortie audio doivent être comprimées est un format de codage audio avancé (AAC).

6. Procédé selon l'une quelconque des revendications 1 à 6, dans lequel le format dans lequel les données d'entrée audio sont comprimées est un format de codage audio avancé (AAC), le format dans lequel les données de sortie audio doivent être comprimées est un format de codage arithmétique à bit tranché (BSAC), et le format de codage audio avancé (AAC) partage une partie des informations latérales des canaux droit et gauche.

7. Procédé selon la revendication 5 ou 6, dans lequel un standard auquel appartient le format AAC est soit un standard MPEG-2 soit un standard MPEG-4.

8. Procédé selon la revendication 5, 6 ou 7, dans lequel un standard auquel appartient le format BSAC est un standard MPEG-4.

9. Procédé selon la revendication 1, dans lequel le format dans lequel sont comprimées les données d'entrée audio est un format de codage audio avancé (AAC), le format
dans lequel les données de sortie audio doivent être comprimées est un format de codage arithmétique à bit tranché (BSAC), et le format de codage audio avancé (AAC) ne partage aucune partie des informations latérales des canaux droit et gauche.

10. Dispositif de conversion des données audio comprenait :
une unité de décodage (300) agencée pour décoder sans perte les données d'entrée audio conformément à un format
dans lequel sont comprimées les données d'entrée audio ; et
une unité de codage (302) agencée pour coder sans perte les résultats décodés sans perte conformément à un format dans lequel les données de sortie audio doivent être comprimées ;
dans lequel l'unité de décodage (300) comprend :
une partie d'éclatement des données (320) agencée pour obtenir des informations latérales des données d'entrée audio ; et
une partie de décodage sans perte (322) agencée pour décoder sans perte les données d'entrée audio à l'exception des informations latérales conformément au format dans lequel sont comprimées les données d'entrée audio et la sortie des résultats décodés sans perte en tant que données quantifiées,
dans lequel l'unité de codage (302) comprend :
une partie de codage sans perte (368) agencée pour coder sans perte les données quantifiées conformément au format dans lequel doivent être comprimées les données de sortie audio ; et
une partie de combinaison des données (370) agencée pour combiner les résultats codés sans perte avec les informations latérales pour générer les données de sortie audio.
dans lequel l'unité de décodage comprend en outre au moins l'une des parties suivantes ;
une partie de quantification inverse (324) agencée pour quantifier inversement l'entrée de données quantifiées provenant de l'unité de décodage sans perte ;
une première partie de traitement stéréo (326) agencée pour traiter les résultats inversement quantifiées ;
une première partie de mise en forme temporelle du bruit (TNS) (328) qui soumet au traitement TNS les résultats du traitement stérée ; et
une première partie de conversion de domaine (330) agencée pour convertir les données situées dans le domaine fréquence en tant que résultats du traitement TNS en données dans le domaine temps,
dans lequel l'unité de codage (302) comprend en outre au moins l'une des parties suivantes :
une deuxième partie de conversion de domaine (360) agencée pour convertir les données situées dans le domaine temps en données dans le domaine fréquence ;
une deuxième partie TNS (362) agencée par TNS pour traiter les données dans le domaine fréquence ;
une deuxième partie de traitement stéréo (364) agencée pour soumettre au traitement stéréo les résultats du traitement TNS ; et
une partie de quantification (366) agencée pour quantifier les résultats du traitement stéréo obtenus par la deuxième partie de traitement stéréo et agencée pour fournir les résultats quantifiés à la partie de codage sans perte,
dans lequel la partie de codage sans perte (368) est agencée pour coder sans perte les résultats quantifiés obtenus par la partie de quantification conformément au format dans lequel les données de sortie audio doivent être comprimées, et
**caractérisé sen ce qu**'il comprend en outre une unité de vérification agencée pour déterminer si une partie des informations latérales des canaux droit et gauche est partagée et en ce qu'il fournit le résultat de la détermination,
dans lequel, en réponse aux résultats vérifiés, la ou les parties de quantification inverse (324), la première partie de traitement stéréo (326), la première partie TNS (328), la première partie de conversion de domaine (330), et la ou les deuxièmes parties de conversion de domaine (360), la deuxième partie TNS (362), la deuxième partie de traitement stéréo (364), et la partie de quantification (366) sont agencées pour fonctionner en fonction des résultats vérifiées ;
et en ce que seulement lorsqu'une partie des informations latérales des canaux droit et gauche n'est pas partagée, la première partie de conversion de domaine (330), la première partie TNS (328), la première partie de traitement stéréo (326) et la partie de quantification inverse (324), la deuxième partie de conversion de domaine (360), la deuxième partie TNS (362), la deuxième partie de traitement stéréo (364) et la partie de quantification (366) sont agencées pour fonctionner ;
dans lequel l'unité de codage (302) code les résultats décodés en utilisant seulement les informations latérales de l'un des canaux droit et gauche.

11. Dispositif selon la revendication 10, dans lequel la partie de quantification (366) est agencée pour minimiser le bruit de quantification en utilisant les informations contenues dans lesdites informations obtenues des données d'entrée audio et similaires à une valeur seuil de masquage.

12. Dispositif selon la revendication 10, dans lequel le dispositif fonctionnant conformément à chaque trame est agencé pour fonctionner à partir d'une trame précédente d'une trame actuelle jusqu'à ce qu'apparaisse une trame dans laquelle est partagée une partie des informations latérales des canaux droit et gauche.

13. Dispositif selon la revendication 10, dans lequel le dispositif fonctionnant conformément à chaque trame est agencé pour fonctionner à partir d'une trame actuelle jusqu'à ce qu'apparaisse une trame dans laquelle est partagée une partie des informations latérale des canaux droit et gauche.
